# EUROPEAN PATENT SPECIFICATION

(11) **EP 1 641 358 B1**
(45) Date of publication and mention of the grant of the patent: **10.08.2011**
(21) Application number: 04739789.8
(22) Date of filing: 11.06.2004
(51) Int. Cl.: A23L 1/305, A23L 1/304, A23L 1/29, A23G 3/00

(54) **NUTRITION BAR**
ERNÄHRUNGSRIEGEL
BARRE NUTRITIVE

(30) Priority: 03.07.2003 US 613483
(43) Date of publication of application: 05.04.2006
(73) Proprietor: Unilever N.V., 3013 AL Rotterdam (NL); Unilever PLC, London EC4Y 0DY (GB)
(72) Inventor: PALMER, Alan, Edward UBF Foodsolutions North Ameri, Lisle, Illinois 60532 (US); RUDAN, Brenda, Jean The International Food Network, Road Ithaca, New York 14850 (US)
(74) Representative: Wurfbain, Gilles L.
(86) International application number: PCT/EP2004/006290
(87) International publication number: WO 2005/002366

(56) References cited:
- EP-A- 0 306 773
- EP-A- 1 302 111
- US-A- 4 859 475
- US-A- 4 900 566
- US-A- 5 418 010
- US-A- 5 683 739
- US-A1- 2003 054 088
- US-B1- 6 248 375
- US-B1- 6 444 700

## Description

### Field of the invention

The present invention relates to edible nutrition bars, especially nutrition bars useful in the control or maintenance of body weight.

### Background of the Invention

Increasingly, a focus of modern preventive medicine is weight reduction. Excessive weight is frequently cited in reports concerning the surge in cases of type 2 diabetes. Moreover, obesity is often mentioned in discussions of other modern diseases, such as heart disease.

For years a debate has raged as to which class of nutrients, fats or carbohydrates, are preferentially minimized to promote weight loss. Recently, much consumer attention has focussed on those who advocate reduction of carbohydrates and higher intakes of unsaturated fat and/or protein.

An increasingly popular form for ingestion of nutrients for those seeking to lose weight is the nutrition bar. The nutrition bar provides a convenient vehicle for replacing a meal or for supplementing meals as a snack. While consumers express a preference for snacks and other foods which are more healthful and which can assist them to achieve their weight loss goals, they show little inclination to sacrifice the organoleptic properties of their favorite foods. Therefore, the successful food formulator must improve the nutrient value of the food while maintaining desirable organoleptic properties. High protein levels are particularly difficult to incorporate into good tasting foods since popular proteins, such as soy and/or rice, often have undesirable (after)tastes or develop such undesirable tastes or aftertastes upon storage. In particular, with certain products comprising soy and/or rice proteins an off-flavour may develop upon storage. Also the appearance and/or texture of such foods may deteriorate over time.

EP-A-1,302,111 discloses dietary bar compositions comprising soy protein, and low levels of glycerol. Some of the examples of bar compositions further comprise sorbitol.

Joseph et al., US patent No 5, 389, 395 disclose an unbaked low calorie food bar comprising proteinaceous material which may be soy protein and a digestible carbohydrate which may be sorbitol or glycerin.

Gilles et al. US Patent No. 6,248,375 (Abbott Labs) discloses solid matrix materials designed for the person with diabetes. It includes a source of fructose in combination with at least one nonabsorbent carbohydrate. The two component carbohydrate system is said to blunt the postprandial carbohydrate response. One of the forms for administration mentioned is nutritional bars. Gilles et al. disclose in the examples nutritional bars comprising about 15 or 16 % by weight of soy protein, about 4.6% by weight of glycerin and a vitamin and mineral pre-mix comprising zinc, iron and copper. Choice dm® Bar is cited as a nutritional bar for people with diabetes and including 17.1% total calories as protein in the form of calcium caseinate, soy protein isolate, whey protein concentrate, toasted soybeans, soy nuggets (soy protein isolate, rice flour, malt, salt) and peanut butter. Gluc-O-Bar® is said to be a medical food designed for use in management of diabetes which includes up to 23% of total calories as protein in the form of soy protein isolate, non fat dry milk, and peanut flour. The typical amount of protein in the Gilles bars is about 10% to about 25% of total calories, most preferably about 15 to about 20% of total calories.

Keating et al. EP 768 043 (Bristol Meyers-Squibb) is directed to a nutritional composition for use by diabetics containing a controlled absorbed carbohydrate component. The carbohydrate component contains a rapidly absorbed fraction such as glucose or sucrose, a moderately absorbed fraction such as certain cooked starches or fructose and a slowly absorbed fraction such as raw corn starch. Preferred protein sources are said to include whey protein, sodium caseinate, or calcium caseinate, optionally supplemented with amino acids. Other preferred protein sources include protein hydrolysates such as soy protein hydrolysate, casein hydrolysate, whey protein hydrolysate, other animal and vegetable protein hydrolysates and mixtures thereof. Among the forms mentioned which the invention can take are a nutritional bar or cookie. The nutritional bars and cookies are preferably baked.

WO 01/56402 discloses an alpha lipoic acid food supplement for increasing lean muscle mass and strength in athletes. A source of amino acids is included. Whey protein is said to be a preferred source of amino acids although other proteins which may be used include casein, other milk proteins, and albumins. The food supplements can be in a variety of forms such as protein bars.

Portman US Patent No. 6,051,236 is directed to a nutritional composition in dry powder form for optimizing muscle performance during exercise. The compositions may be in the form of an energy bar. Soy protein mentioned as one of the possible proteins.

Kaufman WO 01/33976 (Children's Research Hospital) is directed to a method for treating a type 2 diabetic to decrease hypoglycemic episodes and/or diminish fluctuations in blood glucose outside of the normal range, which comprises administering to the subject in an effective appetite suppressing amount a food composition, which can be a bar, which includes a slowly absorbed complex carbohydrate such as uncooked cornstarch. Soy protein, whey protein and casein hydrolysate are mentioned as possible protein sources.

DeMichele et al. US Patent No. 6,444,700 (Abbott Labs) is directed to immunonutritional products said to be useful in reducing the immunological suppression said to result from stress. Solid nutritional compositions such as bars are mentioned. Soy proteins are mentioned as possible ingredients for the solid compositions.

Lanter et al. US Patent No. 5,683,739 is directed to extruded animal feed nuggets comprising between about 90 and 99 wt% of at least one protein containing ingredient and between 1 and 6 wt% added fat. The nugget is prepared by plasticizing a blend of at least one protein-containing ingredient, added fat, sulfur (if present), and water, extruding the plasticized blend to form an animal feed nugget, and drying the extruded nugget to a water content of less than about 12 wt%. Protein sources mentioned include oil seed meals such as soybean meal and cottonseed meal, and animal byproduct meals such as meat meal, poultry meal, blood meal, feather meal, and fish meal, plant byproduct meal such as wheat middlings, soybean hulls, and corn byproducts and microbial protein such as torula yeast and brewer's yeast. US Patent Nos. 5,540,932 and 5,120,565 also are directed to animal feed nuggets which contain, or may contain, protein.

Various other foods have been described which mention nuggets which may include meat proteins. These include US Patent Nos. 6,086,941, 6,010,738.

Wang et al. US patent No. 6,379,725 discloses protein based, edible chewable pet toys such as artificial dog bones, which comprise 70-86 wt% protein which is a mixture of plant derived protein and animial derived proteim. Minerals such as iron and zinc may be present in trace concentrations and plasticisers such as glycerol may be used.

Despite the many previous efforts to formulate nutrition bars with high levels of protein there is still a need for a good tasting nutrition bar having elevated levels of protein, especially nutrition bars comprising soy and/or rice protein and desirable levels of certain minerals, especially transition metals. In particular, there is a need for such a nutrition bar which does not develop an off-taste on storage and which has good sensorial properties (in particular which is moist and chewy), even after prolonged storage at elevated temperatures e.g. 30oC for 4 weeks. It is also desirable that the bar retains a pleasing appearance for the consumer upon storage e.g. does not brown or otherwise change colour. These have been found to be particular problems in nutrition bars comprising soy protein and transition metals and/or transition metal compounds.

### Summary of the Invention

The present invention is directed especially to a nutrition bar which incorporates elevated levels of soy and/or rice protein, at least one transition metal or transition metal compound, and about 2%wt or more of a humectant. In the nutrition bar the at least one transition metal or transition metal compound is in a substantially water insoluble form at 20°C, or, the nutrition bar has an Aw of 0.45 or less, or, the nutritional bar comprises 10% wt or more of soy and/or rice protein in the form of nuggets and the humectant is selected from the group consisting of polyols.

Thus according to a first aspect the present invention provides a nutritional bar comprising;
a) 10%wt or more of soy and/or rice protein, in the form of nuggets,
b) at least one transition metal or transition metal compound, and
c) 2%wt or more of a humectant selected from the group
   consisting of polyols.

According to a second aspect the present invention provides a nutritional bar comprising;
a) 10%wt to 40%wt or more of soy and/or rice protein,
b) at least one transition metal or transition metal compound, and
c) 3 to 10%wt of glycerol humectant,
   and wherein the nutrition bar has an Aw of 0.45 or less.

According to a third aspect the present invention provides a nutritional bar comprising;
a) 10%wt to 40%wt of soy and/or rice protein,
b) at least one transition metal or transition metal compound, wherein the at least one transition metal or transition metal compound is in a substantially water insoluble form at 20°C, and
c) 2%wt or more glycerol humectant.

By the above-mentioned features of the invention, the nutrition bars are formulated to comprise elevated levels of protein yet do not suffer unacceptably from a deterioration in taste or other organoleptic properties such as appearance (e.g. browning) or texture over time. It is preferred that the bars of the invention do not suffer from the aforementioned problems for at least 6 months upon storage at 20oC, more preferably at least 7 months, most preferably at least 8 months, ideally at least one year.

Nuggets as used according to the present invention can have a variety of cross sections, e.g., circular, rectangular or square, and generally are bite sized particles having a maximum volume of 35mm³ and a minimum volume of 4 mm³, preferably between 10mm³ and 25mm³. The soy and/or rice protein nuggets referred to herein will often comprise additional ingredients, such as a reducing sugar, in addition to the soy and/or rice protein.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material or conditions of reaction, physical properties of materials and/or use are to be understood as modified by the word "about." All amounts are by weight, based on the total weight of the relevant product, unless otherwise specified.

Unless stated otherwise or required by context, the terms "fat" and "oil" are used interchangeably herein.

Unless stated otherwise or required by context, the terms "nutritional bar(s)" and "nutrition bar(s)" are used interchangeably herein.

Unless stated otherwise, all percentages are by weight based on the total weight of the composition.

For a more complete explanation of the above and other features and advantages of the invention, reference should be made to the following description of the preferred embodiments. The preferred embodiments apply to all aspects of the invention and can be used as appropriate for each aspect.

### Detailed Description of the Invention

### Protein

The nutritional bars of the invention comprise about 10%wt or more in total of soy and/or rice protein based on the total weight of the composition. It is preferred that the nutritional bars comprise 12%wt to 40%wt, e.g. 12%wt to 35%wt of soy and/or 9 rice protein, more preferably 13%wt to 30%wt, most preferably 14%wt to 25%wt based on the total weight of the composition.

The soy protein may be present in any suitable form including as isolated soy protein, as soy protein concentrate or as soy protein hydrolysates. Sources of rice protein include rice flour and rice protein concentrate

Without wishing to be bound by theory, it is believed that soy and/or rice protein based nutritional bars may suffer from problems of off-flavour development etc because of the presence of free amino acid groups.

According to the first aspect of the invention, the nutritional bars comprise 10%wt or more soy and/or rice protein based on the total weight of the composition, in the form of nuggets (hereinafter protein nuggets). For the other aspects of the invention this is preferred. It is preferred that the nutritional bars comprise 10%wt to 25%wt soy and/or rice protein in the form of nuggets, especially 10%wt to 20%wt. It is preferred that 80%wt or more of the soy and/or rice protein in the bar is present in the form of nuggets, more preferably 90%wt or more, most preferably 95%wt or more, such as 100%wt.

The protein nuggets preferably comprise 50%wt to 100%wt of soy and/or rice protein, more preferably 55%wt to 100%wt, most preferably 60%wt to 95%wt, such as 75%wt to 95%wt based on the weight of the protein nuggets.

The protein nuggets may also comprise one or more of other proteins, such as those listed below, lipids, especially triglyceride fats, and carbohydrates, especially starches. It is especially preferred that the protein nuggets further comprise from 1%wt to 40%wt of a reducing sugar, more preferably 2%wt to 25%wt, most preferably 3%wt to 20%wt. It is advisable that the remaining ingredients be no more sensitive to heat degradation (e.g. have the same or lower degradation point) than the soy and/or rice protein.

In addition to the soy and/or rice protein, other types of protein may also be included in the nutritional bars. Preferred sources for the other protein which may be used in the present invention (either within the protein nugget or within the bar external to the nugget) include dairy protein sources such as whole milk, skim milk, condensed milk, evaporated milk, milk solids non-fat, etc., and include whey protein such as whey protein isolate and whey protein concentrate and caseins; sources of pea protein; and sources of gelatin protein. The amounts of the other proteins, when present, are preferably within the range of from 1%wt to 10%wt, preferably 2%wt to 5%wt.

Especially preferred, to minimize the caloric impact, is the addition of protein as such rather than as one component of a food ingredient such as whole milk. Preferred in this respect are protein concentrates such as one or more of whey protein concentrate, milk protein concentrate, caseinates such as sodium and/or calcium caseinate.

Total protein levels (soy and/or rice and other protein) within the nutrition bars of the invention, including any protein present in the form of nuggets, are preferably within the range of 10wt% to 40 wt%, especially from 12%wt to 40%wt, more preferably 13%wt to 30%wt, most preferably 14%wt to 25%wt based on the total weight of the composition.

The total protein present in the nutritional bar preferably provides up to 50% of the total calories of the bar, more preferably between 20 % and 50%, most preferably between 25% and 50%.

The present invention may also be applied to milk protein based nutrition bars if the same problems as outlined hereinabove are found in these bars.

### Transition metals and transitional metal compounds

According to all aspects of the invention the nutrition bar comprises at least one transition metal or transition metal compound. The transition metal is preferably selected from chromium, manganese, iron, cobalt, nickel, copper, zinc, and mixtures thereof. The transition metal compounds are preferably compounds of these transition metals. It has been found that iron, cobalt, nickel, copper and zinc can cause particular taste and sensorial problems in nutrition bars comprising soy and/or rice proteins.

According to the third aspect of the invention, the at least one transition metal or transition metal compound is in a substantially water insoluble form at 20°C and this is preferred for the other aspects of the invention. The transition metal or transition metal compound may be provided in the substantially water insoluble form by any suitable means. It is preferred that either a substantially water insoluble salt is used, or, that the metal or compound is substantially encapsulated in a suitable encapsulant is used to achieve the desired level of water insolubility.

It is advisable to ensure that the transition metal or transition metal compound is in a substantially water insoluble form at all processing temperatures to which the nutrition bar is subjected during its preparation and ideally also at 5°C or more above the maximum temperature reached during preparation.

Any substantially water insoluble compound of a transition metal may be used according to the invention, especially substantially water insoluble inorganic compounds. Such compounds selected from oxides, carbonates and phosphates (including pyrophosphates) are preferred. If copper is used then copper carbonate is preferred. If iron is used then ferric pyrophosphate is preferred. If zinc is used then zinc oxide is preferred.

The nutrition bars of the invention, typically overall comprise up to 100%, typically up to 50%, such as 10 to 35% of the European 2003 RDA of the transition metal. The exact amount of the transition metal and/or transition metal compound will depend upon the type used. Typically the nutrition bars, per bar, will comprise one or more of up to 1 mg of manganese, up to 1.1 mg of copper, up to 9.5 mg of zinc and up to 16 mg of iron. More preferably the nutrition bars comprise one or more of up to 0.5 mg of manganese, up to 0.4 mg of copper, up to 3 mg of zinc and up to 5 mg of iron.

Alternatively, or additionally, the transition metal or transition metal compound may be encapsulated to render it substantially water insoluble. This provides a wider choice of the types of transition metal compound which may be used and may allow the inclusion of a more bioavailable compound to be used. Any suitable encapsulant may be used. It is especially preferred that an encapsulant is used which does not allow any significant water transmission across the encapsulation layer at temperatures below the melting point of the encapsulant. This is especially important where the encapsulated transition metal or transition metal compound is subjected to elevated temperatures, e.g. of 60°C or more during the preparation of the nutrition bar.

The term "encapsulated" refers both to an embodiment wherein a coating is substantially formed around the transition metal or transition metal compound and to an embodiment wherein the transition metal or transition metal compound is trapped within or throughout a matrix so that it is rendered substantially water insoluble. The transition metal or transition metal compound preferably has a substantially integral encapsulant coating or matrix around it.

Suitable encapsulant materials include substantially water insoluble edible waxes, proteins, fibres, carbohydrates. The encapsulant material may be cross-linked.

Proteins which may be used as the entire encapsulant material, or as a part thereof, include gelatin, milk proteins (including caseinates, such as sodium caseinate, and whey proteins such as beta-lactoglobulin and alpha lactalbumin), albumin and vegetable proteins including proteins from beans, legumes and cereals such as soy, pea, maize and wheat and isolated soy proteins.

Carbohydrates which may be used as the entire encapsulant material, or as a part thereof, include mono or polysaccharides including, cellulose polymers and starches, (including hydrolysed and modified starches) and sugar alcohols. Suitable materials include gum arabic, carrageenan, agar agar, alginates, pectins and pectates.

Preferred encapsulants are carbohydrates such as alginates or pectins, especially including the sodium, potassium and calcium salts of alginates.

Mixtures of sodium caseinate and either gum arabic, carrageenan, agar agar, and gum arabic, are suitable. Similarly, beta-lactoglobulin and either gum arabic, carrageenan, agar agar, alginate or pectins, especially beta-lactoglobulin and gum arabic may be used.

It is preferred that the weight ratio of the transition metal and/or transition metal compound to the encapsulant is in the range of from 5:1 to 1:15, preferably 1:2 to 1:12, e.g. 1:5 to 1:10.

The transition metal or transition metal compound may be encapsulated by any suitable encapsulation technique as known in the art, such as coacervation or spraying on, and does not require further explanation here.

By the term "substantially water insoluble" is meant that the transition metal or transition metal compound does not substantially dissolve in water, in particular that it has a solubility in water at 20°C of 1g/100g deionised water or less, preferably 0.5g/100g deionised water or less.

### Humectant

The nutrition bars according to all aspects of the invention comprise 2%wt or more of a humectant. For the first aspect of the invention the bars comprise 2% or more of a humectant selected from the group consisting of polyols. For the second and third aspects of the invention the bars comprise 3 to 10%wt and 2%wt or more glycerol humectant respectively.

Any suitable humectant, and mixtures thereof, may be used for the second and third aspects of the invention. However, for all aspects it is preferred that the humectant is selected from polyols, with diols and triols being preferred, most especially triols. Suitable diols include sugar alcohol diols. Suitable triols include sugar alcohol triols, glycerol and sorbitol. For all aspects of the invention, especially good results have been obtained when the humectant comprises glycerol, in particular when the nutrition bars comprise 3 to 10% weight glycerol, especially 4 to 7%wt glycerol.

Other humectants which may be used include fruit pastes such as raisin paste, prune pastes or date paste.

It is preferred that the nutrition bars comprise from 3%wt to 15%wt of humectants, more preferably 3%wt to 15%wt, especially 3%wt to 10%wt.

### Aw

According to the second aspect of the invention, the nutrition bar has an Aw of 0.45 or less. This is also preferred for the other aspects of the invention. For all aspects of the invention, it is preferred that the nutrition bar has an Aw of 0.43 or less, most preferably of 0.40 or less. The determination of the Aw is within the normal skill of the skilled person and does not need to be described further here.

### Fat/Carbohydrate

The source for any fat used in the nutrition bars, whether internal or external to the protein nugget, is preferably vegetable fat, such as for example, cocoa butter, illipe, shea, palm, palm kernal, sal, soybean, safflower, cottonseed, coconut, rapeseed, canola, corn and sunflower oils, or mixtures thereof. However, animal fats such as butter fat may also be used if consistent with the desired nutritional profile of the product. Preferably the amount of fat in either the protein nugget or the bar as a whole, is not more than 45 wt%, especially not more than 35 wt%, preferably from 0.5 to 20 wt%, still preferably from 1 to 15 wt%.

Carbohydrates can be used within the protein nuggets at levels of from 1%wt to about 35wt% of the protein nuggets. In addition to sweeteners mentioned below, examples of suitable carbohydrates include starches such as are contained in rice flour, flour, tapioca flour, tapioca starch, and whole wheat flour and mixtures thereof. Carbohydrates can be used outside the protein nuggets within the bar as well. Levels of carbohydrates in the bar as a whole will typically comprise from 5 wt% to 80 wt%, especially from 20% to 65 wt%, such as from 25% to 60 wt%.

### Optional ingredients

If it is desired to include a bulking agent in the nutrition bars, within or external to the protein nuggets, a preferred bulking agent is inert polydextrose. Other conventional bulking agents which may be used alone or in combination therewith include maltodextrin, sugar alcohols, corn syrup solids, sugars or starches. Total bulking agent levels in the protein nuggets, and in the nutritional bars of the invention, will preferably be from about 0% to 20 wt%, preferably 5% to 16%. Polydextrose may be obtained under the brand name Litesse.

Flavorings are preferably added to the nutrition bar in amounts that will impart a mild, pleasant flavor. The flavoring may be present in any protein nuggets or external to the nuggets in the bar, provided that processing is not adversely affected. The flavoring may be any of the commercial flavors typically employed in nutrition bars, such as varying types of cocoa, pure vanilla or artificial flavor, such as vanillin, ethyl vanillin, chocolate, malt, mint, yogurt powder, extracts, spices, such as cinnamon, nutmeg and ginger, mixtures thereof, and the like. It will be appreciated that many flavor variations may be obtained by combinations of the basic flavors. The nutrition bars are flavored to taste and suitable amounts of each flavouring agent desired will therefore be included. Suitable flavorants may also include seasoning, such as salt, and imitation fruit or chocolate flavors either singly or in any suitable combination. Flavorings which mask off-tastes from vitamins and/or minerals and other ingredients are preferably included in the products of the invention, in the protein nuggets and/or elsewhere in the product.

The protein nuggets and/or nutrition bar may include colorants, if desired, such as caramel colorant or vegetable or fruit colourings.

If desired, the protein nuggets and/or nutrition bar may include processing aids such as calcium chloride.

The nutritional bars may comprise one or more cholesterol lowering agents in conventional amounts. Any suitable, known, cholesterol lowering agent may be used, for example isoflavones, phytosterols, soy bean extracts, fish oil extracts, tea leaf extracts.

The food product may optionally comprise, in suitable amounts, one or more agents which may beneficially influence (postprandial) energy metabolism and substrate utilisation, for example caffeine, flavonoids (including tea catechins, capsaicinoids and canitine).

The protein nuggets and/or nutrition bar may also include emulsifiers. Typical emulsifying agents may be phospholipids and proteins or esters of long chain fatty acids and a polyhydric alcohol. Lecithin is an example. Fatty acid esters of glycerol, polyglycerol esters of fatty acids, sorbitan esters of fatty acids and polyoxyethylene and polyoxypropylene esters of fatty acids may be used but organoleptic properties, or course, must be considered. Mono- and di-glycerides are preferred. If present in the nuggets, emulsifiers may be used in amounts of about 0.03% to 0.3%, preferably 0.05% to 0.1%. The same emulsifiers may also be present in the nutrition bar, again at levels overall of about 0.03% to 1%, preferably 0.05% to 0.7%. Emulsifiers may be used in combination, as appropriate.

Among fiber sources which may be included in the nutrition bars of the invention are fructose oligosaccharides such as inulin, soy fiber, fruit fibre e.g. apple, guar gum, gum arabic, gum acacia, oat fiber, cellulose and mixtures thereof. The compositions preferably contain at least 2 grams of fiber per 56 g serving, especially at least 5 grams of fiber per serving. Preferably, fiber sources are present in the product at greater than 0.5 wt% and do not exceed 15 wt%, especially 10 wt%. As indicated above, additional bulking agents such as maltodextrin, sugar alcohols, corn syrup solids, sugars, starches and mixtures thereof may also be used. Total bulking agent levels in the products of the invention, including fibers and other bulking agents, will preferably be from about 0% to 20%, especially from 1 to 15 wt%. The fiber and the bulking agent may be present in the protein nuggets or in the bar external to the nuggets provided that processing is not impaired.

Carrageenan may be included in the bars of the invention, internal or external to any protein nuggets, eg, as a thickening and/or stabilizing agent. Cellulose gel and pectin are other thickeners which may be used alone or in combination.

Generally the nutrition bars of the invention will be naturally sweetened. The sweetener may be included in any protein nuggets provided that it does not interfere with the processing of the nugget (eg, sweetener will not be used in the nugget if it is unstable at the moderate temperatures, where the moderate temperature extrusion process is used) or it may be used in the bar. Natural sources of sweetness include sucrose (liquid or solids), glucose, fructose, and corn syrup (liquid or solids), including high fructose corn syrup and high maltose corn syrup and mixtures thereof. Other sweeteners include lactose, maltose, glycerine, brown sugar and galactose and mixtures thereof. Levels of sugars and sugar sources preferably result in sugar solids levels of up to 50 wt%, preferably from 5 to 18 wt%, especially from 10 to 17 wt% of the nutrition bar.

If it is desired to use artificial sweeteners, these may likewise be present in any protein nuggets or within the bar external to the nugget, provided that it does not interfere with processing. Any of the artificial sweeteners well known in the art may be used, such as aspartame, saccharine, Alitame® (obtainable from Pfizer), acesulfame K (obtainable from Hoechst), cyclamates, neotame, sucralose, mixtures thereof and the like. The sweeteners are used in varying amounts of about 0.005% to 1wt% on the bar, preferably 0.007% to 0.73% depending on the sweetener, for example. Aspartame may be used at a level of 0.05% to 0.15%, preferably at a level of 0.07% to 0.11%. Acesulfame K is preferred at a level of 0.09% to 0.15%.

Calcium is preferably present in the nutrition bars at from 10 to 30% USRDA, especially about 25% USRDA. The calcium source is preferably dicalcium phosphate. For example wt% levels of dicalcium phosphate may range from 0.5 to 1.5%. In a preferred embodiment, the product is fortified with one or more vitamins and/or minerals (in addition to those referred to above in the first to third aspects of the invention) and/or fiber sources, in addition to the calcium source. These may include any or all of the following:
Ascorbic acid (Vitamin C), Tocopheryl Acetate (Vitamin E), Biotin (Vitamin H), Vitamin A Palmitate, Niacinamide (Vitamin B3), Potassium Iodide, d-Calcium Pantothenate (Vitamin B5), Cyanocobalamin (Vitamin B12), Riboflavin (Vitamin B2), Thiamine Mononitrate (Vitamin B1), Molybdenum, Selenium, Calcium Carbonate, Calcium Lactate, Magnesium (e.g., as magnesium phosphate).

One or more of these vitamins and minerals are preferably present at from 5 to 45% USRDI for 2003, especially 5 to 20% RDI, most especially from about 15% RDI.

It is especially preferred that the nutritional bars comprise at least 300 mg of potassium per serving, more preferably 400-1000, most preferably 450-700mg.

The vitamins and/or minerals may be included within, or external to, any protein nuggets, provided that processing and human absorption are not impaired.

Ingredients which, if present, will generally be found within the bar but external to any protein nuggets include, but are not limited to, rolled oats, chocolate chips or other chocolate pieces, cookie and/or cookie dough pieces, such as oatmeal cookie pieces, brownie pieces, fruit pieces, such as dried cranberry, apple, etc., vegetable pieces such as rice, honey and acidulants such as malic and citric acids, leavening agents such as sodium bicarbonate and peanut butter.

The nutritional bars preferably have a calorie content in the range of from 50 kilocalories (kcals) to 250 kcals, more preferably 75 kcals to 200 kcals, most preferably 100 or 150 kcals to 400 kcals per bar.

A single serving size of the nutrition bar is typically in the range of from 45g to 70g, especially 50g to 65g, such as 55g to 60g.

### Manufacture of bars

The nutritional bars may be made by known methods provided that any protein nuggets are not exposed to temperatures which cause degradation of their ingredients, especially, the proteins or encapsulant if present.

Extruded nutritional bars may be made by cooking a syrup containing liquid (at ambient temperature) ingredients and then mixing with dry ingredients. The mixture is then extruded onto a conveyor belt and cut with a cutter. Any protein nuggets are included among the dry ingredients and should only be added to the syrup when the syrup is at a temperature below that at which any of the nugget components degrade. Supercritical fluid extrusion of the bar as a whole at reduced temperatures can also be considered. Syrup ingredients may include components such as corn syrup, glycerine, lecithin and soybean oil or other liquid oils. In addition to any protein nuggets, other dry components include grains, flours, maltodextrin and milk powders may be used.

Nutritional bars in the form of granola bars may be made by cooking the syrup, adding the dry ingredients, blending the syrup and dry ingredients in a blender, feeding the blended mix through rollers and cutting with a cutter.

The bars of the invention may be fully or partially coated, e.g. with milk chocolate or yogurt flavored coating. Suitable conventional coating methods may be used.

Typically, excluding moisture lost during processing, uncoated bars of the invention will be made from 30-70 wt% syrup, especially 35-65%, and 70-30 wt% dry ingredients, especially 65-35 wt%.

The nutritional bar is preferably one intended to be used as part of a weight loss or weight control plan.

Alternative forms of the nutritional bars are powders, tablets and non-bar meal replacement products. The disclosures herein are equally applicable to these other product forms.

A meal replacement product as referred to herein refers to a product which is intended to replace one or more conventional meals per day; they are of a controlled calorie content and are generally eaten as a single product. Examples of meal replacement products include; liquid products such as milk or soya-based drinks, soluble powders used to prepare those drinks and drinks prepared therefrom, bars, soups, cereal or noodle or pasta-based products, desserts such as rice puddings, custards and the like. Meal replacement products are generally used by consumers following a calorie controlled diet. The nutritional bars of the invention may also be consumed as meal replacement products.

The invention will be further illustrated by reference to the following examples. Further examples within the scope of the invention will be apparent to the person skilled in the art.

### Example 1

Two Granola-style nutrition bars were made to the following compositions:

| | A | B |
|---|---|---|
| | %wt | %wt |
| **Binder:** | | |
| Glucose syrup | 8.903 | 11.861 |
| Polydextrose syrup | 9.90 | 10.0 |
| Inulin syrup | 4.6 | 4.6 |
| Sugar | 2.3 | 2.3 |
| Pectose paste | 5.0 | 5.0 |
| Coconut oil | 2.3 | 2.3 |
| Lecithin | 0.6 | 0.6 |
| Glycerol | 5.0 | 1.242 |
| Invert syrup | 4.9 | 4.9 |
| Date paste | 3.0 | 3.0 |
| Corn oil | 2.1 | 2.1 |
| Flavourings | 0.375 | 0.375 |
| Colourings | 0.144 | 0.144 |
| Water loss | -3.20 | -3.20 |

| **Dry material:** | | |
|---|---|---|
| Oatflakes | 4.324 | 5.5 |
| Coconut flakes, sweetened and shredded | 2.2 | 2.2 |
| Fruit fibre | 4.15 | 4.15 |
| Soy protein nuggets | 6.0 | 6.0 |
| 1^{*1} | | |
| Soy protein nuggets 2^{*2} | 23.5 | 23.1 |
| Vitamin/mineral mix ^{*3} | 3.904 | 3.904 |

| **Coating:** | | |
|---|---|---|
| Dairy coating | 10.00 | 10.00 |
| A_{W} | 0.40 +/- 0.03 | 0.53 +/- 0.02 |
| Bar weight | 60.0 g | 60.0 g |

| | | |
|---|---|---|
| *1 - soy protein nuggets comprising 60%wt soy protein, available from Dupont Protein Technologies Inc., USA. *2 - soy protein nuggets comprising 80%wt soy protein, available from Dupont Protein Technologies Inc., USA. *3 - vitamin/mineral mix comprising zinc, iron, copper. In bar A there was 2mg of zinc, 1mg of iron and 0.18mg of copper. In bar B there was 3mg of zinc, 4.9mg of iron and 0.34mg of copper. For bar A, insoluble copper carbonate encapsulated with sodium alginate (1:9 weight ratio) was used. For bar B, soluble copper gluconate encapsulated with hardened soybean oil was used. | | |

Bar A was prepared by the following method of preparation; The glucose syrup, polydextrose syrup, inulin syrup, sugar, Pectose paste, coconut oil and lecithin, were heated together to about 250oF, 86.5 Brix and moisture loss recorded. The glycerol was added with mixing. Separately the invert syrup and date paste were mixed together and heated to 230oF whereafter the mixture was added to the glycerol-containing mixture with stirring. The mixture was allowed to cool to 180oF when the corn oil was added with mixing. After further cooling to 140oF, the flavours and colourings were added. The dry materials were mixed separately and added to the cooled mixture above with mixing until a uniform mixture was formed. Bars were formed by pressing the mixture into a mould, and when cooled to room temperature, cutting the cooled mixture into dimensions of 11cm x 3.5cm x 1.9cm.

The bar was coated with the dairy coating which was allowed to set.

Bar B was prepared by the method for bar A except the first heating stage was to 225-230oF, 83 Brix, that invert syrup was added with the other binder ingredients and the date paste was added with the colourings and flavouring. The bar was cut into dimension of 11cm x 3.5cm x 1.9cm.

### Results

The nutrition bars were stored under accelerated storage conditions at either 30oC or 36oC, or, normal storage at 20oC to assess them for off flavour development and a deterioration in the organoleptic properties. Bar A was stable after 4 months accelerated storage at 30oC which is the equivalent of more than 12 months storage at 20oC, showing no unacceptable off-flavour development and no unacceptable deterioration in other organoleptic properties. The bars were still chewy, moist and with a good taste after 12 months storage at 20oC. No unacceptable browning of the bar was observed. Bar B was stable for only 4 weeks at 36oC and 6 months at 20oC but thereafter quickly produced a nutty off-flavour and browning.

It should be understood of course that the specific forms of the invention herein illustrated and described are intended to be representative only, as certain changes may be made therein without departing from the clear teaching of the disclosure. Accordingly, reference should be made to the appended claims in determining the full scope.

## Claims

1. A nutritional bar comprising;
a) 10%wt or more of soy and/or rice protein in the form of nuggets,
b) at least one transition metal or transition metal
compound, and
2%wt or more of a humectant selected from the group consisting of polyols.

2. A nutritional bar according to claim 1 comprising 12%wt to 35%wt of soy and/or rice protein.

3. A nutritional bar according to any one of claims 1 to 2 wherein the nuggets comprise 50%wt to 100%wt of soy and/or rice protein.

4. A nutritional bar according to claim 3 wherein the nuggets comprise 75%wt to 95%wt of soy and/or rice protein.

5. A nutritional bar according to any one of the preceding claims wherein the nuggets further comprise 2%wt to 25%wt of a reducing sugar.

6. A nutritional bar according to any one of the preceding claims wherein the polyol is selected from the group consisting of triols.

7. A nutritional bar according to claim 6 wherein the triol comprises glycerol.

8. A nutritional bar according to any one of the preceding claims comprising 3%wt to 20%wt of the humectant.

9. A nutritional bar according to any one of the preceding claims wherein the humectant comprises 3%wt to 10%wt of
glycerol.

10. A nutritional bar according to any one of the preceding claims wherein the at least one transition metal or transition metal compound is selected from the group consisting of chromium, manganese, iron, cobalt, nickel, copper, zinc, and their compounds and mixtures thereof.

11. A nutritional bar according to any one of the preceding claims wherein the at least one transition metal or transition metal compound is in a substantially water insoluble form at 20°C.

12. A nutritional bar according to claim 11 wherein the at least one transition metal or transition metal compound is substantially encapsulated in an encapsulation material.

13. A nutritional bar comprising;
a) 10%wt to 40%wt of soy and/or rice protein,
b) at least one transition metal or transition metal compound, and
c) 3 to 10%wt of glycerol humectant,
and wherein the nutrition bar has an Aw of 0.45 or less.

14. A nutritional bar according to claim 13 wherein the nutritional bar has an Aw of 0.43 or less.

15. A nutritional bar comprising;
a) 10%wt to 40%wt of soy and/or rice protein,
b) at least one transition metal or transition metal compound, wherein the at least one transition metal or transition metal compound is in a substantially water insoluble form at 20°C, and
c) 2%wt or more of glycerol humectant.

16. A nutritional bar according to claim 15 wherein the at least one transition metal or transition metal compound is substantially encapsulated in an encapsulation material.

## Patentansprüche

1. Ernährungsriegel, umfassend:
a) 10 Gewichts-% oder mehr Soja- und/oder Reisprotein in Form von Klümpchen,
b) wenigstens ein Übergangsmetall oder eine Übergangsmetallverbindung und 2 Gewichts-% oder mehr eines Feuchthaltemittels, das aus der Gruppe, bestehend aus Polyolen, ausgewählt ist.

2. Ernährungsriegel gemäß Anspruch 1, der 12 Gewichts-% bis 35 Gewichts-% Soja- und/oder Reisprotein umfasst.

3. Ernährungsriegel gemäß einem der Ansprüche 1 bis 2, wobei die Klümpchen 50 Gewichts-% bis 100 Gewichts-% Soja- und/oder Reisprotein umfassen.

4. Ernährungsriegel gemäß Anspruch 3, wobei die Klümpchen 75 Gewichts-% bis 95 Gewichts-% Soja- und/oder Reisprotein umfassen.

5. Ernährungsriegel gemäß einem der vorangehenden Ansprüche, wobei die Klümpchen außerdem 2 Gewichts-% bis 25 Gewichts-% eines reduzierenden Zuckers umfassen.

6. Ernährungsriegel gemäß einem der vorangehenden Ansprüche, wobei das Polyol aus der Gruppe, bestehend aus Triolen, ausgewählt ist.

7. Ernährungsriegel gemäß Anspruch 6, wobei das Triol Glycerin umfasst.

8. Ernährungsriegel gemäß einem der vorangehenden Ansprüche, der 3 Gewichts-% bis 20 Gewichts-% des Feuchthaltemittels umfasst.

9. Ernährungsriegel gemäß einem der vorangehenden Ansprüche, wobei das Feuchthaltemittel 3 Gewichts-% bis 10 Gewichts-% Glycerin umfasst.

10. Ernährungsriegel gemäß einem der vorangehenden Ansprüche, wobei das wenigstens eine Übergangsmetall oder die wenigstens eine Übergangsmetallverbindung aus der Gruppe, bestehend aus Chrom, Mangan, Eisen, Kobalt, Nickel, Kupfer, Zink und deren Verbindungen und Gemischen davon, ausgewählt ist.

11. Ernährungsriegel gemäß einem der vorangehenden Ansprüche, wobei das wenigstens eine Übergangsmetall oder die wenigstens eine Übergangsmetallverbindung bei 20 °C in einer im Wesentlichen wasserunlöslichen Form ist.

12. Ernährungsriegel gemäß Anspruch 11, wobei das wenigstens eine Übergangsmetall oder die wenigstens eine Übergangsmetallverbindung in einem Einkapselungsmaterial im Wesentlichen eingekapselt ist.

13. Ernährungsriegel, umfassend:
a) 10 Gewichts-% bis 40 Gewichts-% Soja- und/oder Reisprotein,
b) wenigstens ein Übergangsmetall oder eine Übergangsmetallverbindung und
c) 3 bis 10 Gewichts-% Glycerin-Feuchthaltemittel,
und wobei der Ernährungsriegel einen Aw-Wert von 0,45 oder weniger hat.

14. Ernährungsriegel gemäß Anspruch 13, wobei der Ernährungsriegel einen Aw-Wert von 0,43 oder weniger hat.

15. Ernährungsriegel, umfassend:
a) 10 Gewichts-% bis 40 Gewichts-% Soja- und/oder Reisprotein,
b) wenigstens ein Übergangsmetall oder eine Übergangsmetallverbindung, wobei das wenigstens eine Übergangsmetall oder die wenigstens eine Übergangsmetallverbindung bei 20 °C in einer im Wesentlichen wasserunlöslichen Form ist, und
c) 2 Gewichts-% oder mehr Glycerin-Feuchthaltemittel.

16. Ernährungsriegel gemäß Anspruch 15, wobei das wenigstens eine Übergangsmetall oder die wenigstens eine Übergangsmetallverbindung in einem Einkapselungsmaterial im Wesentlichen eingekapselt ist.

## Revendications

1. Barre nutritive comprenant :
a) 10 % en poids ou plus de protéine de soja et/ou de riz sous la forme de pépites,
b) au moins un métal de transition ou composé de métal de transition, et
2 % en poids ou plus d'un humidifiant choisi dans le groupe constitué par les polyols.

2. Barre nutritive selon la revendication 1 comprenant 12 à 35 % en poids de protéine de soja et/ou de riz.

3. Barre nutritive selon l'une quelconque des revendications 1 à 2, dans laquelle les pépites comprennent de 50 à 100 % en poids de protéine de soja et/ou de riz.

4. Barre nutritive selon la revendication 3, dans laquelle les pépites comprennent de 75 à 95 % en poids de protéine de soja et/ou de riz.

5. Barre nutritive selon l'une quelconque des revendications précédentes, dans laquelle les pépites comprennent, en outre, de 2 à 25 % en poids d'un sucre réducteur.

6. Barre nutritive selon l'une quelconque des revendications précédentes, dans laquelle le polyol est choisi dans le groupe constitué par les triols.

7. Barre nutritive selon la revendication 6, dans laquelle le triol comprend le glycérol.

8. Barre nutritive selon l'une quelconque des revendications précédentes comprenant de 3 à 20 % en poids d'humidifiant.

9. Barre nutritive selon l'une quelconque des revendications précédentes, dans laquelle l'humidifiant comprend de 3 à 10 % en poids de glycérol.

10. Barre nutritive selon l'une quelconque des revendications précédentes, dans laquelle ledit au moins un métal de transition ou composé de métal de transition est choisi dans le groupe constitué par le chrome, le manganèse, le fer, le cobalt, le nickel, le cuivre, le zinc, et leurs composés et mélanges.

11. Barre nutritive selon l'une quelconque des revendications précédentes, dans laquelle ledit au moins un métal de transition ou composé de métal de transition est sous une forme essentiellement insoluble dans l'eau à 20°C.

12. Barre nutritive selon la revendication 11, dans laquelle ledit au moins un métal de transition ou composé de métal de transition est essentiellement encapsulé dans un matériau d'encapsulation.

13. Barre nutritive comprenant :
a) de 10 à 40 % en poids de protéine de soja et/ou de riz,
b) au moins un métal de transition ou composé de métal de transition, et
c) de 3 à 10 % en poids d'un humidifiant de
type glycérol,
ladite barre nutritive ayant une activité en eau (Aw) de 0,45 ou moins.

14. Barre nutritive selon la revendication 13, dans laquelle la barre nutritive a une activité en eau (Aw) de 0,43 ou moins.

15. Barre nutritive comprenant :
a) de 10 à 40 % en poids de protéine de soja et/ou de riz,
b) au moins un métal de transition ou composé de métal de transition, ledit au moins métal de transition ou composé de métal de transition étant sous une forme essentiellement insoluble dans l'eau à 20°C, et
c) 2 % en poids ou plus d'un humidifiant de type glycérol.

16. Barre nutritive selon la revendication 15, dans laquelle ledit au moins un métal de transition ou composé de métal de transition est essentiellement encapsulé dans un matériau d'encapsulation.
